# EUROPEAN PATENT APPLICATION

(11) **EP 3 135 371 A1**
(43) Date of publication of application: **01.03.2017**
(21) Application number: 15782614.0
(22) Date of filing: 06.02.2015
(51) Int. Cl.: B01J 23/745, B01J 23/83, B01J 23/889, B01J 23/75, B01J 23/86, B01J 23/80, B01J 23/89, B01J 23/78, C10G 2/00

(54) **STRUCTURED IRON-BASED CATALYST FOR PRODUCING A-OLEFIN FROM SYNTHESIS GAS AND PREPARATION METHOD AND USE**

(30) Priority: 21.04.2014 CN 201410160961
(71) Applicant: Wuhan Kaidi Engineering Technology Research Institute Co., Ltd., Wuhan, Hubei 430223 (CN)
(72) Inventor: CHEN,Yilong, Wuhan Hubei 430212 (CN); ZHANG, Yanfeng, Wuhan Hubei 430212 (CN); CHEN, Jiangang, Wuhan Hubei 430212 (CN); SONG, Dechen, Wuhan Hubei 430212 (CN); ZHANG, Juan, Wuhan Hubei 430212 (CN); SUN, Taomei, Wuhan Hubei 430212 (CN)
(74) Representative: Zeuner Summerer Stütz
(86) International application number: PCT/CN2015/072400
(87) International publication number: WO 2015/161700

(57) **Abstract**

A structured iron-based catalyst for producing an α-olefin from a synthesis gas and a preparation method and use. The catalyst comprises an active component iron, auxiliary agents and a carrier, wherein the auxiliary agents comprise a first auxiliary agent which is a transition metal or a transition metal oxide and a second auxiliary agent which is a metal oxide. The content of the active component iron is 50.0%-99.8%, the content of the first auxiliary agent is 0-5.0%, the content of the second auxiliary agent is 0-10% and the balance is the carrier which is silicon dioxide. A precursor of the active component iron, a precursor of the first auxiliary agent and the carrier silicon dioxide are made into mono-dispersed particles using a heat dispersing method, and then impregnated with a solution of a precursor of the second auxiliary agent to obtain the structured iron-based catalyst. The above-mentioned structured iron-based catalyst is used for producing an α-olefin from a synthesis gas. Since the mono-dispersed structured iron-based catalyst can generate an active centre suitable for the generation of the α-olefin, it thus has good selectivity.

## Description

### TECHNICAL FIELD

The invention relates to a catalyst and a method for preparing the catalyst, and an application of the catalyst; more particularly, the invention relates to a structured iron-based catalyst, a method for preparing the catalyst, and a use of the catalyst for producing α-olefin.

### DESCRIPTION OF THE RELATED ART

Long chain α-olefin refers to the mono-olefin or the mixed olefin with an unsaturated bond at a position between the fourth carbon number and the sixteenth carbon number on the chain hydrocarbon end. Because of the active site (the olefinic bond), the α-olefin can be connected to various functional groups to form compounds such as alcohol, acid, ester. Thus the α-olefin is widely applied to the production of fine chemicals such as surfactant and plasticizer. At present, the α-olefin is mainly used as a commoner to produce polyethylene, accounting for 44.1 % of the total consumption. The most widely used types of the α-olefin as the commoner are C4(1-butene), C6(1-hexene), and C8(1-octene), which are applied to the production of high-density polyethylene or liner low-density polyethylene (HDPE/LLDPE) to improve the polyethylene density, the tear resistance, and tensile strength. Meanwhile, the α-olefin can be applied to the higher alcohol production, which is used to produce plasticizer and detergent, accounting for 19.6% of the total consumption. The higher alcohol is the basic material for preparing surfactant and plasticizer, and a consumption thereof worldwide reaches ten million tons per year. Octanol is mainly used to prepare esters, and the sales of which is more than a billion dollars worldwide each year. Heptanol can be used to prepare flavor and perfume, thus the heptanol is valuable. The α-olefin is also used to prepare poly-α-olefin (PAO), accounting for 16.6% of the total consumption. PAO is the high-end lubricant base oil with a high viscosity index and a price twice or triple the price of mineral oil. In addition, the α-olefin is the material for preparing emulsifier, leather treatment agent, lubricant additive, antirust agent, fabric finishing agent, paper, and so on.

Foreign total productive capacity of α-olefin is about 2.12 million tons, and the productive capacity of α-olefin in China is 260 thousand tons. In addition, China is incapable of producing long chain α-olefin, and the 1-hexene and the 1-octene completely rely on import.

Conventional method for preparing α-olefin uses the paraffin cracking process or the ethylene oligomerization process. The paraffin cracking process generally uses deoiling refined wax as the material. The wax is preheated and is mixed with steam to perform a reaction in the tubular cracking furnace at about 550°C. The ethylene oligomerization process uses the triethyl aluminum as the catalyst; a controlled chain growth reaction of the ethylene is performed, and the olefin having certain chain length is polymerized. Specifically, there are two-step method, one-step method, and SHOP process for preparing the α-olefin.

Conventional α-olefin preparation is based on wax or ethylene, and the resources are derived from petroleum. However, the oil and gas resources are declining, and the decline of oil and gas resources worldwide makes the energy resources become more diversified. The non-oil route using clean liquid fuel and chemicals which are originated from coal, biomass, and natural gas by forming the syngas is drawing attention.

The disclosed Chinese patents which relate to a method for preparing olefin are classified according to the difference in core technology as follows:

Technology 1: The Chinese patent No. 101265151 disclosed a method for preparing light olefin using methanol or dimethyl ether. The methanol or dimethyl ether as the material is transmitted to the reaction zone of the reactor via the feed distributor, and reacts in the presence of the molecular sieve catalyst to yield a mixture of light olefin, diolefin, oxygenated chemicals, and C₄ hydrocarbons.

Technology 2: The Chinese patent No. 101265149 and the Chinese patent No. 1537674 disclosed methods for preparing ethylene, propylene, butylene using syngas in the presence of iron/activated carbon catalyst, which are characterized in the specific chemical composition of the self-made catalyst.

Technology 3: The Chinese patent No. 1444551, the Chinese patent No. 1545520, the Chinese patent No. 1443149, and the Chinese patent No. 1440311 disclosed methods for preparing linear α-olefin and a catalyst system thereof.

Technology 4: The Chinese patent No. 1403425 and the Chinese patent No. 1421424 disclosed methods and devices for preparing higher carbon α-olefin using high alkyl aluminum. The methods use a paraffin cracking process.

Technology 5: The Chinese patent No. 1284898A and Chinese patent No. 1515359A disclosed methods for preparing iron-based catalyst particles using a self-supporting precipitation process at a slurry bed.

### SUMMARY OF THE INVENTION

In view of the above-described problems, it is one objective of the invention to provide a structured iron-based catalyst, a method for preparing the catalyst, and an application of the catalyst for producing α-olefin.

To achieve the above objective, in accordance with one embodiment of the invention, there is provided a structured iron-based catalyst for α-olefin preparation, comprising between 50.0 and 99.8 percent by weight of iron, between 0 and 5.0 percent by weight of a first additive, between 0 and 10 percent by weight of a second additive, and between 0 and 50 percent by weight of a carrier. The first additive is transitional metal ruthenium, platinum, copper, cobalt, or zinc of group VIB, VIIB, or VIII; or the first additive is transitional-metal oxide. The second additive is lanthanum oxide, cerium oxide, magnesium oxide, aluminum oxide, silicon oxide, potassium oxide, manganese oxide, or zirconium oxide. The carrier is silicon dioxide. The iron, the first additive, and the carrier are mono-dispersed particles prepared using a thermal diffusion method, and the second additive is adapted to soak the mono-dispersed particles.

In a class of this embodiment, optionally, a content of the first additive is between 0 and 2%. A content of the second additive being metal oxide is between 2% and 6%. A content of the iron is between 60% and 97%.

In a class of this embodiment, optionally, a content of the carrier being the silicon dioxide is between 1% and 40%. A content of the first additive is between 1% and 2%. A content of the second additive being metal oxide is between 2% and 6%. A content of the iron is between 52% and 96%.

A method for preparing the structured iron-based catalyst, comprising:
1) Monodispersed catalyst precursor A preparation using thermal decomposition method:
   mixing iron nitrate having crystal water removed, nitrate of the first additive, and amorphous silicon dioxide with n-octanol to form a mixed solution, where a total mass percentage of the iron nitrate, the nitrate of the first additive, and the amorphous silicon dioxide in the mixed solution is between 3% and 20%; stirring the mixed solution so that the nitrate is dissolved and heating the mixed solution to a temperature between 140 and 180°C; keeping the temperature constant for 4 hrs; cooling and filtrating the mixed solution to yield a first mixture; drying the first mixture to yield black solid; grinding the black solid for 20 to 40 mins using a planetary mill, then roasting the black solid in a muffle furnace for 5 hrs at between 400 and 600°C to yield a catalyst precursor A;
2) dissolving the second additive in water or ethyl alcohol to form a second additive solution; dry impregnating the catalyst precursor A in the second additive solution and aging for between 12 and 24 hrs to form a second mixture; drying the second mixture at a temperature between 100 and 130°C, and roasting the second mixture for 4 to 10 hrs at a temperature between 300 and 1200°C; compressing the second mixture to be tablets and sieving to yield the structured iron-based catalyst.

In a class of this embodiment, a structured iron-based catalyst comprises between 1 and 40 percent by weight of the carrier, between 1 and 2 percent by weight of the first additive, between 2 and 6 percent by weight of the second additive, and between 52 and 96 percent by weight of the iron.

In a class of this embodiment, the iron nitrate having crystal water removed, the nitrate of the first additive, and the amorphous silicon dioxide are mixed with n-octanol to form the mixed solution in 1) of the method, where a total mass percentage of the iron nitrate, the nitrate of the first additive, and the amorphous silicon dioxide in the mixed solution is between 5% and 15%

In a class of this embodiment, a particle size of the catalyst precursor A is between 50 and 60 nm. The catalyst precursor is spherical, and is monodispersed.

An application of the catalyst for producing α-olefin, comprising a Fischer-Tropsch synthesis reaction at a fixed bed using syngas. A reducing condition thereof is: 300-500°C, 0.2-1.2 MPa, 400-1500 h-1 (VN), 6-18 hrs, and under pure hydrogen atmosphere. A syngas reaction condition is: 190-360°C, 0.5-5.0 MPa, 400-20000 h-1 (V/V), and H2/CO=1/1-3/1.

The application of the catalyst for producing α-olefin comprises a Fischer-Tropsch synthesis reaction at a slurry bed using syngas. A reducing condition thereof is: 300-500°C, 0.2-1.2 MPa, 400-1400 rpm, 400-1500 h-1 (V/V), 6-18 hrs, and under pure hydrogen atmosphere. A syngas reaction condition is: 190-360°C, 0.5-5.0 MPa, 400-20000 h-1 (V/V), 400-1400 rpm, and H2/CO=1/1-3/1.

Advantages of the structured iron-based catalyst, the method for preparing the catalyst, and the application thereof according to embodiments of the invention are summarized as follows:

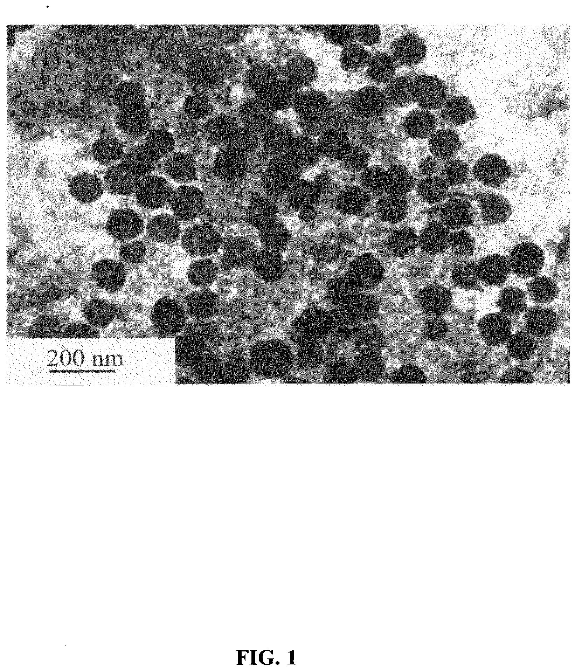

1. Compared with the conventional method, the thermal decomposition method in the invention is used to prepare the catalyst with regular shape, uniform pore size, and particular microstructure. As shown in FIG. 1, the particles of the iron-based catalyst are uniform, spherical, and monodispersed. The particle size is about 70 nm. The structure of the catalyst ensures the active centers to be uniform and facilitates the formation of active centers. Meanwhile, as the catalyst powders have uniform shapes, the pore structures are more ideal. All of the above lead to high α-olefin selectivity of the catalyst.

2. Because strengths of the iron and the additive are properly arranged, the catalyst features high reductivity and high degree of carbonization. By choosing from the types of carriers, adjusting the dosage, and using the additive, the strengths of the iron and the carrier are properly arranged, thus preventing sintering of the iron component, and avoiding components which are difficult to be reduced. The catalyst features favorable chemical properties. Due to the synergistic effect of the structure and chemical properties of the catalyst, the catalyst features high long chain α-olefin selectivity and activity.

3. Optionally, the catalyst is compressed to be tablets, or the catalyst is shaped via spray drying. The catalyst features favorable mechanical properties.

A novel method is provided in the invention for producing α-olefin. The method uses syngas in the presence of the catalyst to directly synthesize α-olefin. The materials, the target product, the technical route, and the method for preparing the catalyst in the invention are different from those in the prior art, thus the new technology for preparing α-olefin is totally different from the existing technology, and involves in an inventive step. Because of the monodispersed structure and the appropriate meta-carrier chemical effect, the catalyst features the appropriate active centers for α-olefin generation, and the catalyst features favorable selectivity.

### BRIEF DESCRIPTION OF THE DRAWINGS

FIG. 1 is a transmission electron microscopy (TEM) photo of an iron-based catalyst prepared using a thermal decomposition method of an exemplary embodiment of the invention.

### DETAILED DESCRIPTION OF THE EMBODIMENTS

### Example 1

60 g of iron nitrate having crystal water removed and 10 g of amorphous silicon dioxide were mixed with 800 mL of n-octanol to form a mixed solution. The mixed solution was stirred so that the nitrate was dissolved, and the mixed solution was heated to 140°C. The temperature was maintained for 4 hrs. The mixed solution was cooled and filtrated to yield a first mixture. The first mixture was dried to yield black solid. The black solid was grinded using a planetary mill, then the black solid was roasted in a muffle furnace for 5 hrs at 400°C to yield a catalyst precursor. 0.55 g of La(NO₃)₃ was dissolved in water to form 18 mL of a second additive solution. The catalyst precursor was soaked in the second additive solution and was aged for 12 hrs to form a second mixture. The second mixture was dried at 100°C, and was roasted for 10 hrs at 800°C. The second mixture was compressed to be tablets and sieved to yield the structured iron-based catalyst 53%Fe1%La₂O₃/ SiO₂ comprising 53 percent by weight of iron, 1 percent by weight of La₂O₃, and 53 percent by weight of silicon dioxide.

1.5 mL of the catalyst having the particle sizes of between 60 and 80 meshes was added in a pressurized fixed bed reactor (*Φ*10×500 mm), in which the catalyst was heated at a programmed temperature and was reduced under pure hydrogen atmosphere. The reducing condition was: 400°C, 0.2 MPa, 400h⁻¹ (V/V), and 18 hrs. Following the reduction, the reactor was cooled, and the syngas was introduced to perform a syngas reaction. The syngas reaction condition was: 230°C, 2.5 MPa, 2000 h⁻¹ (V/V), and H₂/CO=2/1. The result of the reaction is shown in Table 2.

15 mL of the catalyst having the particle size being above 140 meshes was added in a slurry agitator with a volume of 1 L, then 500 mL of liquid paraffin was added in the slurry agitator to form a third mixture. The third mixture was heated at a programmed temperature and was reduced under pure hydrogen atmosphere. The reducing condition was: 300°C, 0.2 MPa, 400 h⁻¹ (V/V), 400 rpm, and 18 hrs. Following the reduction, the reactor was cooled, and the syngas was introduced to perform a syngas reaction. The syngas reaction condition was: 240°C, 0.5 MPa, 3000 h⁻¹ (V/V), 400 rpm, and H₂/CO=2/1. The result of the reaction is shown in Table **2.**

### Example 2

80 g of iron nitrate having crystal water removed and 5 g of amorphous silicon dioxide were mixed with 800 mL of n-octanol to form a mixed solution. The mixed solution was stirred so that the nitrate was dissolved, and the mixed solution was heated to 140°C. The temperature was maintained for 4 hrs. The mixed solution was cooled and filtrated to yield a first mixture. The first mixture was dried to yield black solid. The black solid was grinded using a planetary mill, then the black solid was roasted in a muffle furnace for 5 hrs at 400°C to yield a catalyst precursor. 0.55 g of K₂CO₃ was dissolved in water to form 18 mL of a second additive solution. The catalyst precursor was soaked in the second additive solution and was aged for 12 hrs to form a second mixture. The second mixture was dried at 100°C, and was roasted for 10 hrs at 400°C. The second mixture was compressed to be tablets and sieved to yield the structured iron-based catalyst 75%Fe2%K₂O1%MnO/SiO₂ comprising 75 percent by weight of iron, 2 percent by weight of K₂O, 1 percent by weight of MnO, and the rest is silicon dioxide.

1.5 mL of the catalyst having the particle sizes of between 60 and 80 meshes was added in a pressurized fixed bed reactor (*Φ*10×500 mm), in which the catalyst was heated at a programmed temperature and was reduced under pure hydrogen atmosphere. The reducing condition was: 400°C, 0.4 MPa, 800 h⁻¹ (VN), and 12 hrs. Following the reduction, the reactor was cooled, and the syngas was introduced to perform a syngas reaction. The syngas reaction condition was: 200°C, 1.0 MPa, 1500 h⁻¹ (VN), and H₂/CO=3/1. The result of the reaction is shown in Table 2.

15 mL of the catalyst having the particle size being above 140 meshes was added in a slurry agitator with a volume of 1 L, then 500 mL of liquid paraffin was added in the slurry agitator to form a third mixture. The third mixture was heated at a programmed temperature and was reduced under pure hydrogen atmosphere. The reducing condition was: 400°C, 0.4 MPa, 600 h⁻¹ (VN), 600 rpm, and 12 hrs. Following the reduction, the reactor was cooled, and the syngas was introduced to perform a syngas reaction. The syngas reaction condition was: 200°C, 1.0 MPa, 2000 h⁻¹ (VN), 600 rpm, and H₂/CO=3/1. The result of the reaction is shown in Table **2.**

### Example 3

60 g of iron nitrate having crystal water removed, 10 g of cobalt nitrate, and 5 g of amorphous silicon dioxide were mixed with 800 mL of n-octanol to form a mixed solution. The mixed solution was stirred so that the nitrate was dissolved, and the mixed solution was heated to 140°C. The temperature was maintained for 4 hrs. The mixed solution was cooled and filtrated to yield a first mixture. The first mixture was dried to yield black solid. The black solid was grinded using a planetary mill, then the black solid was roasted in a muffle furnace for 5 hrs at 400°C to yield a catalyst precursor. 1.5 g of Cu(NO₃)₂ was dissolved in water to form 18 mL of a second additive solution. The catalyst precursor was soaked in the second additive solution and was aged for 12 hrs to form a second mixture. The second mixture was dried at 100°C, and was roasted for 10 hrs at 400°C. The second mixture was compressed to be tablets and sieved to yield the structured iron-based catalyst 60%Fe10%Co2%CuO/SiO₂ comprising 60 percent by weight of iron, 10 percent by weight of Co, 2 percent by weight of CuO, and the rest is silicon dioxide.

15 mL of the catalyst having the particle size being above 140 meshes was added in a slurry agitator with a volume of 1 L, then 500 mL of liquid paraffin was added in the slurry agitator to form a third mixture. The third mixture was heated at a programmed temperature and was reduced under pure hydrogen atmosphere. The reducing condition was: 400°C, 0.8 MPa, 600 h⁻¹ (VN), 1000 rpm, and 10 hrs. Following the reduction, the reactor was cooled, and the syngas was introduced to perform a syngas reaction. The syngas reaction condition was: 220°C, 2.0 MPa, 2000 h⁻¹ (V/V), 1000 rpm, and H₂/CO=1/1. The result of the reaction is shown in Table **2.**

### Example 4

60 g of iron nitrate having crystal water removed, 4 g of cobalt nitrate, and 5 g of amorphous silicon dioxide were mixed with 800 mL of n-octanol to form a mixed solution. The mixed solution was stirred so that the nitrate was dissolved, and the mixed solution was heated to 140°C. The temperature was maintained for 4 hrs. The mixed solution was cooled and filtrated to yield a first mixture. The first mixture was dried to yield black solid. The black solid was grinded using a planetary mill, then the black solid was roasted in a muffle furnace for 5 hrs at 400°C to yield a catalyst precursor. 3 g of Zr(NO₃)₄ was dissolved in water to form 18 mL of a second additive solution. The catalyst precursor was soaked in the second additive solution and was aged for 12 hrs to form a second mixture. The second mixture was dried at 100°C, and was roasted for 10 hrs at 600°C. The second mixture was compressed to be tablets and sieved to yield the structured iron-based catalyst 54%Fe5%Co4%ZrO₂/SiO₂ comprising 54 percent by weight of iron, 5 percent by weight of Co, 4 percent by weight of ZrO₂, and the rest is silicon dioxide.

1.5 mL of the catalyst having the particle sizes of between 60 and 80 meshes was added in a pressurized fixed bed reactor (*Φ*10×500 mm), in which the catalyst was heated at a programmed temperature and was reduced under pure hydrogen atmosphere. The reducing condition was: 400°C, 1.2 MPa, 1000 h⁻¹ (VN), and 10 hrs. Following the reduction, the reactor was cooled, and the syngas was introduced to perform a syngas reaction. The syngas reaction condition was: 240°C, 3.0 MPa, 800 h⁻¹ (VN), and H₂/CO=2/1. The result of the reaction is shown in Table **2.**

15 mL of the catalyst having the particle size being above 140 meshes was added in a slurry agitator with a volume of 1 L, then 500 mL of liquid paraffin was added in the slurry agitator to form a third mixture. The third mixture was heated at a programmed temperature and was reduced under pure hydrogen atmosphere. The reducing condition was: 400°C, 0.4 MPa, 600 h⁻¹ (VN), 600 rpm, and 12 hrs. Following the reduction, the reactor was cooled, and the syngas was introduced to perform a syngas reaction. The syngas reaction condition was: 200°C, 1.0 MPa, 2000 h⁻¹ (VN), 600 rpm, and H₂/CO=3/1. The result of the reaction is shown in Table **2.**

### Example 5

80 g of iron nitrate having crystal water removed, and 1 g of amorphous silicon dioxide were mixed with 800 mL of n-octanol to form a mixed solution. The mixed solution was stirred so that the nitrate was dissolved, and the mixed solution was heated to 140°C. The temperature was maintained for 4 hrs. The mixed solution was cooled and filtrated to yield a first mixture. The first mixture was dried to yield black solid. The black solid was grinded using a planetary mill, then the black solid was roasted in a muffle furnace for 5 hrs at 400°C to yield a catalyst precursor. 5 g of Al(NO₃)₃ was dissolved in water to form 18 mL of a second additive solution. The catalyst precursor was soaked in the second additive solution and was aged for 12 hrs to form a second mixture. The second mixture was dried at 100°C, and was roasted for 10 hrs at 800°C. The second mixture was compressed to be tablets and sieved to yield the structured iron-based catalyst 90%Fe4% Al₂O₃/SiO₂ comprising 90 percent by weight of iron, 4 percent by weight of Al₂O₃, and 6 percent by weight of silicon dioxide.

1.5 mL of the catalyst having the particle sizes of between 60 and 80 meshes was added in a pressurized fixed bed reactor (*Φ*10×500 mm), in which the catalyst was heated at a programmed temperature and was reduced under pure hydrogen atmosphere. The reducing condition was: 500°C, 1.2 MPa, 1500 h⁻¹ (V/V), and 6 hrs. Following the reduction, the reactor was cooled, and the syngas was introduced to perform a syngas reaction. The syngas reaction condition was: 260°C, 5.0 MPa, 400 h⁻¹ (V/V), and H₂/CO=2/1. The result of the reaction is shown in Table 2.

15 mL of the catalyst having the particle size being above 140 meshes was added in a slurry agitator with a volume of 1 L, then 500 mL of liquid paraffin was added in the slurry agitator to form a third mixture. The third mixture was heated at a programmed temperature and was reduced under pure hydrogen atmosphere. The reducing condition was: 500°C, 1.2 MPa, 1500 h⁻¹ (V/V), 1400 rpm, and 6 hrs. Following the reduction, the reactor was cooled, and the syngas was introduced to perform a syngas reaction. The syngas reaction condition was: 260°C, 4.0 MPa, 700 h⁻¹ (V/V), 1400 rpm, and H₂/CO=2/1. The result of the reaction is shown in Table **2.**

### Examples 6-10

The examples follow a basic method in the Example 5, except that the content and the additive are different. The contents of the components in the examples are shown in Table **1.** The content of the silicon dioxide equals to the total content of components minus the contents of the three components in Table 1.

**Table 1 Contents of components in the examples**

| Number | Content of iron | Content of the first additive | Content of the second additive |
|---|---|---|---|
| Example 1 | 53%Fe | 1%La₂O₃ | |
| Example 2 | 75%Fe | 2%K₂O | 1%MnO |
| Example 3 | 60%Fe | 10%Co | 2%CuO |
| Example 4 | 54%Fe | 5%Co | 4%ZrO₂ |
| Example 5 | 90%Fe | 6%Al₂O₃ | |
| Example 6 | 80%Fe | 3%Cr₂O₃ | 0.5%K₂O |
| Example 7 | 95%Fe | 1%ZnO | 0.2%K₂O |
| Example 8 | 70%Fe | 2%CeO₂ | 0.3%CuO |
| Example 9 | 68%Fe | 2%TiO₂ | 0.1%Ru |
| Example 10 | 98%Fe | 1%Al₂O₃ | 0.2%K₂O |

**Table 2 Performance of structured iron-based catalyst in olefin synthesis reaction**

| Catalyst | Reaction Condition | CO conversion rate % | C₁ selectivity % | C₅⁺ selectivity % | Olefin content % |
|---|---|---|---|---|---|
| Example 1 | 230°C, 2000 h⁻¹, fixed bed | 42.5 | 6.2 | 75.1 | 61.2 |
| | 240°C, 3000 h⁻¹, slurry bed | 33.6 | 5.5 | 76.6 | 63.8 |
| Example 2 | 250°C, 1500 h⁻¹, fixed bed | 51.2 | 4.8 | 78.0 | 64.4 |
| | 260°C, 2000 h⁻¹, slurry bed | 40.8 | 5.2 | 77.6 | 65.1 |
| Example 3 | 240°C, 10000 h^{-1'}, fixed bed | 12.1 | 14.8 | 58.4 | 45.7 |
| | 220°C, 2000 h⁻¹, slurry bed | 35.9 | 12.4 | 61.3 | 40.6 |
| Example 4 | 240°C, 800 h⁻¹, fixed bed | 66.7 | 9.1 | 74.0 | 58.3 |
| | 260°C, 7000 h⁻¹, slurry bed | 19.6 | 9.4 | 76.4 | 72.1 |
| Example 5 | 260°C, 400 h⁻¹, fixed bed | 33.4 | 15.7 | 47.8 | 46.1 |
| | 260°C, 700 h⁻¹, slurry bed | 38.4 | 15.8 | 46.5 | 35.9 |
| Example 6 | 260°C, 12000h⁻¹, fixed bed | 26.3 | 4.7 | 81.1 | 64.2 |
| | 230°C, 1000 h⁻¹, slurry bed | 52.5 | 4.5 | 81.4 | 63.9 |
| Example 7 | 240°C, 6000 h⁻¹, fixed bed | 28.5 | 6.7 | 78.8 | 65.4 |
| | 250°C, 3000 h⁻¹, slurry bed | 43.3 | 6.1 | 79.5 | 65.9 |
| Example 8 | 280°C, 4000 h⁻¹, fixed bed | 71.2 | 5.2 | 81.3 | 61.4 |
| | 220°C, 1000 h⁻¹, slurry bed | 10.5 | 4.1 | 83.8 | 62.7 |
| Example 9 | 240°C, 2000 h⁻¹, fixed bed | 52.6 | 11.2 | 74.4 | 63.6 |
| Example 10 | 300°C, 4000 h⁻¹, slurry bed | 30.1 | 6.5 | 52.0 | 70.0 |

As shown in FIG. 1, when the contents and the preparation method in the invention are satisfied, monodispersed particles having uniform particle size of 70 nm are prepared. According to the examples, when the contents of the components are not coincident with the method in the invention, for example, the Example 3 and Example 5 have low olefin content; when the contents of the components are coincident with the method in the invention, the example has high olefin content. Within the preferable range of contents, the catalyst features higher α-olefin selectivity.

## Claims

1. A structured iron-based catalyst, comprising:
between 50.0 and 99.8 percent by weight of iron;
between 0 and 5.0 percent by weight of a first additive; the first additive being transitional metal ruthenium, platinum, copper, cobalt, or zinc of group VIB, VIIB, or VIII; or the first additive being transitional-metal oxide.
between 0 and 10 percent by weight of a second additive; the second additive being lanthanum oxide, cerium oxide, magnesium oxide, aluminum oxide, silicon dioxide, potassium oxide, manganese oxide, or zirconium oxide; and
the rest being a carrier; the carrier being silicon dioxide;
wherein
the iron, the first additive, and the carrier are mono-dispersed particles prepared using a thermal diffusion method, and the second additive is adapted to soak the mono-dispersed particles.

2. The catalyst of claim 1, wherein optionally, a content of the first additive is between 0 and 2%; a content of the second additive being metal oxide is between 2% and 6%; and a content of the iron is between 60% and 97%.

3. The catalyst of claim 1, wherein optionally, a content of the carrier being the silicon dioxide is between 1% and 40%; a content of the first additive is between 1% and 2%; a content of the second additive being metal oxide is between 2% and 6%; and the rest is a content of the iron.

4. A method for preparing the catalyst in claims 1-3, comprising:
1) monodispersed catalyst precursor A preparation using thermal decomposition method:
mixing iron nitrate having crystal water removed, nitrate of a first additive, and amorphous silicon dioxide with n-octanol to form a mixed solution; wherein a total mass percentage of the iron nitrate, the nitrate of the first additive, and the amorphous silicon dioxide in the mixed solution is between 3% and 20%; stirring the mixed solution so that the nitrate is dissolved and heating the mixed solution to a temperature between 140 and 180°C; keeping the temperature constant for 4 hrs; cooling and filtrating the mixed solution to yield a first mixture; drying the first mixture to yield black solid; grinding the black solid for 20 to 40 mins using a planetary mill, then roasting the black solid in a muffle furnace for 5 hrs at between 400 and 600°C to yield a catalyst precursor A;
2) dissolving a second additive in water or ethyl alcohol to form a second additive solution; dry impregnating the catalyst precursor A in the second additive solution and aging for between 12 and 24 hrs to form a second mixture; drying the second mixture at a temperature between 100 and 130°C, and roasting the second mixture for 4 to 10 hrs at a temperature between 300 and 1200°C; and compressing the second mixture to be tablets and sieving to yield the structured iron-based catalyst.

5. The method of claim 4, wherein the structured iron-based catalyst comprises between 1 and 40 percent by weight of a carrier being the silicon dioxide, between 1 and 2 percent by weight of the first additive, between 2 and 6 percent by weight of the second additive, and the rest are the iron.

6. The method of claim 4 or 5, wherein the iron nitrate having crystal water removed, the nitrate of the first additive, and the amorphous silicon dioxide are mixed with n-octanol to form the mixed solution in 1) of the method, and a total mass percentage of the iron nitrate, the nitrate of the first additive, and the amorphous silicon dioxide in the mixed solution is between 5% and 15%.

7. The method of claim 4 or 5, wherein a particle size of the catalyst precursor is between 50 and 60 nm; and the catalyst precursor is spherical, and is monodispersed.

8. The method of claim 6, wherein a particle size of the catalyst precursor is between 50 and 60 nm; and the catalyst precursor is spherical, and is monodispersed.

9. A use of the catalyst in any one of claims 4-8 for producing α-olefin, comprising a Fischer-Tropsch synthesis reaction at a fixed bed using syngas; wherein a reducing condition thereof is: 300-500°C, 0.2-1.2 MPa, 400-1500 h⁻¹ (V/V), 6-18 hrs, and under pure hydrogen atmosphere; and a syngas reaction condition is: 190-360°C, 0.5-5.0 MPa, 400-20000 h⁻¹ (V/V), and H₂/CO=1/1-3/1.

10. A use of the catalyst in any one of claims 4-8 for producing α-olefin, comprising a Fischer-Tropsch synthesis reaction at a slurry bed using syngas; wherein a reducing condition thereof is: 300-500°C, 0.2-1.2 MPa, 400-1400 rpm, 400-1500 h⁻¹ (VN), 6-18 hrs, and under pure hydrogen atmosphere; and a syngas reaction condition is: 190-360°C, 0.5-5.0 MPa, 400-20000 h⁻¹ (V/V), 400-1400 rpm, and H₂/CO=1/1-3/1.
